# EUROPÄISCHE PATENTSCHRIFT

(11) **EP 0 920 431 B1**
(45) Veröffentlichungstag und Bekanntmachung des Hinweises auf die Patenterteilung: **26.02.2003**
(21) Anmeldenummer: 97940047.0
(22) Anmeldetag: 30.07.1997
(51) Int. Cl.: C07D 495/04, A61K 31/505

(54) **THIENOPYRIMIDINE**
THIENOPYRIMIDINES
THIENOPYRIMIDINES

(30) Priorität: 12.08.1996 DE 19632423
(43) Veröffentlichungstag der Anmeldung: 09.06.1999
(73) Patentinhaber: MERCK PATENT GmbH, 64293 Darmstadt (DE)
(72) Erfinder: ROCHUS, Jonas, D-64291 Darmstadt (DE); SCHELLING, Pierre, D-64367 Mühltal (DE); KLUXEN, Franz-Werner, D-64285 Darmstadt (DE); CHRISTADLER, Maria, D-63322 Rödermark (DE)
(86) Internationale Anmeldenummer: EP9704139
(87) Internationale Veröffentlichungsnummer: WO98006722

(56) Entgegenhaltungen:
- EP-A- 0 579 496
- EP-A- 0 728 759

## Beschreibung

Die Erfindung betrifft Verbindungen der Formel I 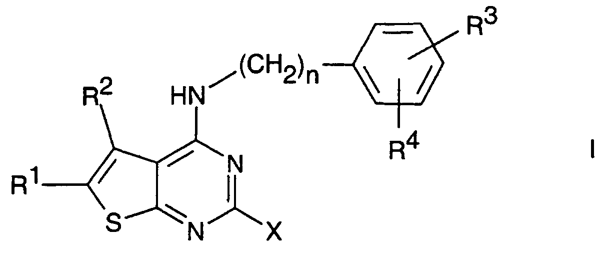 worin
- R¹, R²: jeweils unabhängig voneinander A oder Hal,
- R¹ und R²: zusammen auch Alkylen mit 3-5 C-Atomen,
- R³, R⁴: jeweils unabhängig voneinander H, OA oder Hal,
- R³ und R⁴: zusammen auch -O-CH₂-O- oder -O-CH₂-CH₂-O-,
- A: Alkyl mit 1 bis 6 C-Atomen,
- X: 1-Imidazolyl, 2-Methyl-imidazolyl, Pyrazolyl, 1,2,4-Triazol-1-yl oder 3-Pyridyl,
- Hal: F, Cl, Br oder I
und
- n: 1
bedeuten,
mit der Maßgabe, daß wenn
R¹, R² jeweils unabhängig voneinander A oder Hal
bedeuten,
dann R³ und R⁴ nicht H bedeuten,
sowie deren physiologisch unbedenklichen Salze.

Pyrimidinderivate sind beispielsweise aus der EP 201 188 oder der WO 93/06104 bekannt.
Chinazolinderivate sind aus EP 0579496 bekannt.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze bei guter Verträglichkeit sehr wertvolle pharmakologische Eigenschaften besitzen.

Insbesondere zeigen sie eine spezifische Inhibierung der cGMP-Phosphodiesterase (PDE V).

Chinazoline mit cGMP-Phosphodiesterase hemmender Aktivität sind z.B. in J. Med. Chem. 36, 3765 (1993) und ibid. 37, 2106 (1994) beschrieben.

Die biologische Aktivität der Verbindungen der Formel I kann nach Methoden bestimmt werden, wie sie z.B in der WO 93/06104 beschrieben sind.
Die Affinität der erfindungsgemäßen Verbindungen für cGMP- und cAMP-Phosphodiesterase wird durch die Ermittlung ihrer IC₅₀-Werte (Konzentration des Inhibitors, die benötigt wird, um eine 50 %ige Inhibierung der Enzymaktivität zu erreichen) bestimmt.
Zur Durchführung der Bestimmungen können nach bekannten Methoden isolierte Enzyme verwendet werden (z.B. W.J. Thompson et al., Biochem. 1971, 10, 311). Zur Durchführung der Versuche kann eine modifizierte "batch"-Methode von W.J. Thompson und M.M. Appleman (Biochem. 1979, 18, 5228) angewendet werden.

Die Verbindungen eignen sich daher zur Behandlung von Erkrankungen des Herz-Kreislaufsystems, insbesondere der Herzinsuffizienz und zur Therapie von Potenzstörungen.

Die Verbindungen der Formel I können als Arzneimittelwirkstoffe in der Human- und Veterinärmedizin eingesetzt werden. Ferner können sie als Zwischenprodukte zur Herstellung weiterer Arzneimittelwirkstoffe eingesetzt werden.

Gegenstand der Erfindung sind dementsprechend die Verbindungen der Formel I sowie ein Verfahren zur Herstellung
a) von Verbindungen der Formel I nach Anspruch 1 sowie deren Salzen, worin X über C gebunden ist,
dadurch gekennzeichnet, daß man eine Verbindung der Formel III 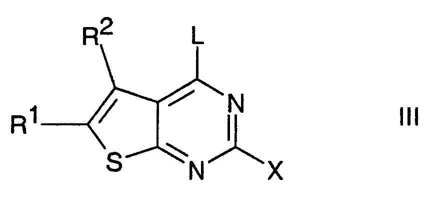 worin
R¹, R² und X die angegebenen Bedeutungen haben,
und L Cl, Br, OH, SCH₃ oder eine reaktionsfähige veresterte OH-Gruppe bedeutet,
mit einer Verbindung der Formel IV 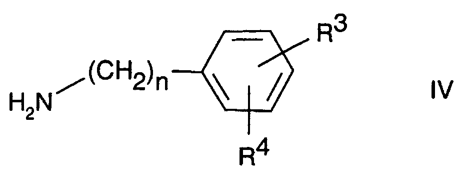 worin
R³, R⁴ und n die angegebenen Bedeutungen haben,
umsetzt,
und/oder daß man eine basische Verbindung der Formel I durch Behandeln mit einer Säure in eines ihrer Salze überführt.

Vor- und nachstehend haben die Reste R¹, R², R³, R⁴, X, L und n die bei den Formeln I, II, III und IV angegebenen Bedeutungen, sofern nicht ausdrücklich etwas anderes angegeben ist.

A bedeutet vorzugsweise Alkyl mit 1-6 C-Atomen.

In den vorstehenden Formeln ist Alkyl vorzugsweise unverzweigt und hat 1, 2, 3, 4, 5 oder 6 C-Atome, vorzugsweise 1, 2, 3, 4 oder 5 C-Atome und bedeutet vorzugsweise Methyl, Ethyl oder Propyl, weiterhin bevorzugt Isopropyl, Butyl, Isobutyl, sek.-Butyl oder tert.-Butyl, aber auch n-Pentyl, neo-Pentyl oder Isopentyl.

Alkylen ist vorzugsweise unverzweigt und bedeutet bevorzugt Propylen, Butylen oder Pentylen.

Ferner bedeuten R¹ und R² auch zusammen bevorzugt Propylen, Butylen oder Pentylen.

Hal bedeutet vorzugsweise F, Cl oder Br, aber auch I.

Die Reste R³ und R⁴ können gleich oder verschieden sein und stehen vorzugsweise in der 3- oder 4-Position des Phenylrings. Sie bedeuten beispielsweise jeweils unabhängig voneinander H, Alkoxy, F, Cl, Br oder I oder zusammen Methylendioxy oder Ethylendioxy. Bevorzugt stehen sie auch jeweils für Alkoxy, wie z.B. für Methoxy, Ethoxy oder Propoxy.

Der Rest X ist vorzugsweise unsubstituiertes 1-Imidazolyl, 2-Methyl-1-imidazol-1-yl, 1-, 3-, 4- oder 5-Pyrazolyl, 3-Pyridyl, weiterhin bevorzugt 1,2,4-Triazol-1-yl.

Für die gesamte Erfindung gilt, daß sämtliche Reste, die mehrfach auftreten, gleich oder verschieden sein können, d.h. unabhängig voneinander sind.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln la bis Ic ausgedrückt werden, die der Formel I entsprechen und worin die nicht näher bezeichneten Reste die bei der Formel I angegebene Bedeutung haben, worin jedoch

| | | |
|---|---|---|
| in Ia | X | Imidazolyl oder Pyridinyl bedeutet; |
| | | |
| in Ib | R¹ und R² | zusammen Alkylen mit 3-5 C-Atomen, |
| | R³ und R⁴ | zusammen -O-CH₂-O- oder -O-CH₂-CH₂-O, |
| | X | Imidazolyl oder Pyridinyl und |
| | n | 1 |

bedeuten;

| | | |
|---|---|---|
| in Ic | R¹ und R² | zusammen Alkylen mit 3-5 C-Atomen, |
| | R³, R⁴ | jeweils unabhängig voneinander H, OA oder Hal, |
| | X | Imidazolyl oder Pyridinyl und |
| | n | 1 |

bedeuten;

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart), beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

In den Verbindungen der Formeln II, III und IV haben R¹, R², R³, R⁴, X und n die angegebenen Bedeutungen, insbesondere die angegebenen bevorzugten Bedeutungen.

Falls L eine reaktionsfähige veresterte OH-Gruppe bedeutet, so ist diese vorzugsweise Alkylsulfonyloxy mit 1-6 C-Atomen (bevorzugt Methylsulfonyloxy) oder Arylsulfonyloxy mit 6-10 C-Atomen (bevorzugt Phenyloder p-Tolylsulfonyloxy, ferner auch 2-Naphthalinsulfonyloxy).

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.
Andererseits ist es möglich, die Reaktion stufenweise durchzuführen.

Die Verbindungen der Formel I, worin X über N an das Thienopyrimidin-Ringsystem gebunden ist, können vorzugsweise erhalten werden, indem man Verbindungen der Formel II 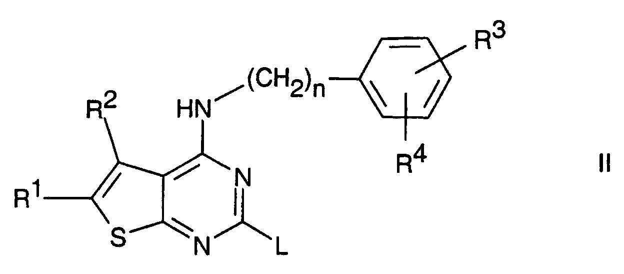 worin
R¹, R², R³, R⁴ und n die angegebenen Bedeutungen haben,
und L Cl, Br, OH, SCH₃ oder eine reaktionsfähige veresterte OH-Gruppe bedeutet, mit einem unsubstituierten oder ein-, zwei- oder dreifach durch A, Hal oder CF₃ substituierten ungesättigten 5-7-gliedrigen Heterocyclus mit mindestens einer NH-Gruppe, worin zusätzlich weitere CH₂-Gruppen durch NH, NA, S oder O ersetzt sein können, umsetzt.

Die Ausgangsstoffe der Formeln II sind teilweise bekannt. Sofern sie nicht bekannt sind, können sie nach an sich bekannten Methoden hergestellt werden.

Vorstufen der Verbindungen der Formel II können z.B. durch Cyclisierung und Halogenierung analog J. Med. Chem. 24, 374 (1981) hergestellt werden. Durch anschließende Umsetzung mit Arylalkylaminen erhält man die Verbindungen der Formel II.

Im einzelnen erfolgt die Umsetzung der Verbindungen der Formel II mit dem NH-haltigen Heterocyclus in Gegenwart oder Abwesenheit eines inerten Lösungsmittels bei Temperaturen zwischen etwa -20 und etwa 150°, vorzugsweise zwischen 20 und 100°.

Der Zusatz eines säurebindenden Mittels, beispielsweise eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums oder Calciums, oder der Zusatz einer organischen Base wie Triethylamin, Dimethylamin, Pyridin oder Chinolin oder eines Überschusses der Aminkomponente kann günstig sein.

Als inerte Lösungsmittel eignen sich z.B. Kohlenwasserstoffe wie Hexan, Petrolether, Benzol, Toluol oder Xylol; chlorierte Kohlenwassertoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Nitroverbindungen wie Nitromethan oder Nitrobenzol; Ester wie Ethylacetat oder Gemische der genannten Lösungsmittel.

Verbindungen der Formel I, worin X über C an das Thienopyrimidin-Ringsystem gebunden ist, können weiterhin erhalten werden, indem man Verbindungen der Formel III mit Verbindungen der Formel IV umsetzt.
Die Ausgangsverbindungen der Formel IV und V sind in der Regel bekannt. Sind sie nicht bekannt, so können sie nach an sich bekannten Methoden hergestellt werden.
Verbindungen der Formel III können z.B. durch Umsetzung mit POCl₃ aus Verbindungen erhalten werden, die aus Thiophenderivaten und CNsubstituierten Heterocyclen aufgebaut werden (Eur. J. Med. Chem. 23, 453 (1988).

Die Umsetzung der Verbindungen der Formel III mit Verbindungen der Formel IV erfolgt unter ähnlichen Bedingungen, betreffend die Reaktionszeit, Temperatur und Lösungsmittel, wie dies für die Umsetzung der Verbindungen der Formel II mit den NH-haltigen Heterocyclen beschrieben ist.

Eine Base der Formel I kann mit einer Säure in das zugehörige Säureadditionssalz übergeführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und Disulfonsäuren, Laurylschwefelsäure. Salze mit physiologisch nicht unbedenklichen Säuren, z.B. Pikrate, können zur Isolierung und /oder Aufreinigung der Verbindungen der Formel I verwendet werden.

Andererseits können, falls gewünscht, die freien Basen der Formel I aus ihren Salzen mit Basen (z.B. Natrium- oder Kaliumhydroxid oder - carbonat) in Freiheit gesetzt werden.

Gegenstand der Erfindung ist ferner die Verwendung der Verbindungen der Formel I und/oder ihrer physiologisch unbedenklichen Salze zur Herstellung pharmazeutischer Zubereitungen, insbesondere auf nicht-chemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.

Gegenstand der Erfindung sind auch Arzneimittel der Formel I und ihre physiologisch unbedenklichen Salze als Phosphodiesterase V-Hemmer.

Gegenstand der Erfindung sind ferner pharmazeutische Zubereitungen, enthaltend mindestens eine Verbindung der Formel I und/oder eines ihrer physiologisch unbedenklichen Salze.

Diese Zubereitungen können als Arzneimittel in der Human- oder Veterinärmedizin verwendet werden. Als Trägerstoffe kommen organische oder anorganische Substanzen in Frage, die sich für die enterale (z.B. orale), parenterale oder topische Applikation eignen und mit den neuen Verbindungen nicht reagieren, beispielsweise Wasser, pflanzliche Öle, Benzylalkohole, Alkylenglykole, Polyethylenglykole, Glycerintriacetat, Gelatine, Kohlehydrate wie Lactose oder Stärke, Magnesiumstearat, Talk, Vaseline. Zur oralen Anwendung dienen insbesondere Tabletten, Pillen, Dragees, Kapseln, Pulver, Granulate, Sirupe, Säfte oder Tropfen, zur rektalen Anwendung Suppositorien, zur parenteralen Anwendung Lösungen, vorzugsweise ölige oder wässrige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und /oder ein oder weitere Wirkstoffe enthalten, z.B. ein oder mehrere Vitamine.

Die Verbindungen der Formel I und ihre physiologisch unbedenklichen Salze können bei der Bekämpfung von Krankheiten, bei denen eine Erhöhung des cGMP(cyclo-Guanosin-monophosphat)-Spiegels zu Entzündungshemmung oder -verhinderung und Muskelentspannung führt, eingesetzt werden. Besondere Verwendung können die erfindungsgemäßen Verbindungen bei der Behandlung von Krankheiten des Herz-Kreislaufsystems und zur Therapie von Potenzstörungen finden.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation.

Massenspektrometrie (MS): EI (Elektronenstoß-lonisation) M⁺
FAB (Fast Atom Bombardment) (M+H)⁺

### Beispiel 1

Eine Lösung von 3,29 g 2,4-Dichlor-6-methyl-thieno-[2,3-d]-pyrimidin in 80 ml Dichlormethan wird mit 3,02 g 3,4-Methylendioxybenzylamin ("A") versetzt und nach Zugabe von 1,52 g Triethylamin 12 Stunden bei Raumtemperatur gerührt. Das Lösungsmittel wird entfernt und wie üblich aufgearbeitet. Man erhält 3,38 g 2-Chlor-6-methyl-4-(3,4-methylendioxybenzylamino)-thieno-[2,3-d]-pyrimidin, F. 162°.

Analog erhält man durch Umsetzung von "A"
mit 2,4-Dichlor-5,6,7,8-tetrahydro-[1]-benzothieno-[2,3-d]-pyrimidin 2-Chlor-5,6,7,8-tetrahydro-4-(3,4-methylendioxybenzylamino)-[1]-benzothieno-[2,3-d]-pyrimidin, F. 222°;
mit 2,4-Dichlor-5,6-cyclopenteno-[1]-benzothieno-[2,3-d]-pyrimidin 2-Chlor-5,6-cyclopenteno-4-(3,4-methylendioxybenzylamino)-thieno-[2,3-d]-pyrimidin;
mit 2,4-Dichlor-5,6-cyclohepteno-[1 ]-benzothieno-[2,3-d]-pyrimidin 2-Chlor-5,6-cyclohepteno-4-(3,4-methylendioxybenzylamino)-thieno-[2,3-d]-pyrimidin;
mit 2,4,5-Trichlor-6-methyl-thieno-[2,3-d]-pyrimidin 2,5-Dichlor-6-methyl-4-(3,4-methylendioxybenzylamino)-thieno-[2,3-d]-pyrimidin;
mit 2,4-Dichlor-5,6-dimethyl-thieno-[2,3-d]-pyrimidin 2-Chlor-5,6-dimethyl-4-(3,4-methylendioxybenzylamino)-thieno-[2,3-d]-pyrimidin.

Analog erhält man durch Umsetzung von 3-Chlor-4-methoxy-benzylamin
mit 2,4-Dichlor-5,6,7,8-tetrahydro-[1]-benzothieno-[2,3-d]-pyrimidin 2-Chlor-5,6,7,8-tetrahydro-4-(3-chlor-4-methoxybenzylamino)-[1]-benzothieno-[2,3-d]-pyrimidin;
mit 2,4-Dichlor-5,6-cyclopenteno-[1]-benzothieno-[2,3-d]-pyrimidin 2-Chlor-5,6-cyclopenteno-4-(3-chlor-4-methoxybenzylamino)-thieno-[2,3-d]-pyrimidin;
mit 2,4-Dichlor-5,6-cyclohepteno-[1]-benzothieno-[2,3-d]-pyrimidin 2-Chlor-5,6-cyclohepteno-4-(3-chlor-4-methoxybenzylamino)-thieno-[2,3-d]-pyrimidin;
mit 2,4,5-Trichlor-6-methyl-thieno-[2,3-d]-pyrimidin 2,5-Dichlor-6-methyl-4-(3-chlor-4-methoxybenzylamino)-thieno-[2,3-d]-pyrimidin;
mit 2,4-Dichlor-5,6-dimethyl-thieno-[2,3-d]-pyrimidin 2-Chlor-5,6-dimethyl-4-(3-chlor-4-methoxybenzylamino)-thieno-[2,3-d]-pyrimidin.

Analog erhält man durch Umsetzung von 3,4-Dimethoxy-benzylamin
mit 2,4-Dichlor-5,6,7,8-tetrahydro-[1]-[1]-benzothieno-[2,3-d]-pyrimidin 2-Chlor-5,6,7,8-tetrahydro-4-(3,4-dimethoxybenzylamino)-[1]-benzothieno-[2,3-d]-pyrimidin;
mit 2,4-Dichlor-5,6-cyclopenteno-[1]-benzothieno-[2,3-d]-pyrimidin 2-Chlor-5,6-cyclopenteno-4-(3,4-dimethoxybenzylamino)-thieno-[2,3-d]-pyrimidin;
mit 2,4-Dichlor-5,6-cyclohepteno-[1]-benzothieno-[2,3-d]-pyrimidin 2-Chlor-5,6-cyclohepteno-4-(3,4-dimethoxybenzylamino)-thieno-[2,3-d]-pyrimidin;
mit 2,4,5-Trichlor-6-methyl-thieno-[2,3-d]-pyrimidin 2,5-Dichlor-6-methyl-4-(3,4-dimethoxybenzylamino)-thieno-[2,3-d]-pyrimidin;
mit 2,4-Dichlor-5,6-dimethyl-thieno-[2,3-d]-pyrimidin 2-Chlor-5,6-dimethyl-4-(3,4-dimethoxybenzylamino)-thieno-[2,3-d]-pyrimidin.

Analog erhält man durch Umsetzung von Benzylamin
mit 2,4-Dichlor-5,6,7,8-tetrahydro-[1]-benzothieno-[2,3-d]-pyrimidin 2-Chlor-5,6,7,8-tetrahydro-4-benzylamino-[1]- benzothieno-[2,3-d]-pyrimidin;
mit 2,4-Dichlor-5,6-cyclopenteno-thieno-[2,3-d]-pyrimidin 2-Chlor-5,6-cyclopenteno-4-benzylamino-thieno-[2,3-d]-pyrimidin;
mit 2,4-Dichlor-5,6-cyclohepteno-thieno-[2,3-d]-pyrimidin 2-Chior-5,6-cyclohepteno-4-benzyiamino-thieno-[2,3-d]-pyrimidin.

Analog erhält man durch Umsetzung von 4-Fluorbenzylamin
mit 2,4-Dichlor-5,6,7,8-tetrahydro-[1]-benzothieno-[2,3-d]-pyrimidin 2-Chlor-5,6,7,8-tetrahydro-4-(4-fluorbenzylamino)-[1]-benzothieno-[2,3-d]-pyrimidin;
mit 2,4-Dichlor-5,6-cyclopenteno-thieno-[2,3-d]-pyrimidin 2-Chlor-5,6-cyclopenteno-4-(4-fluorbenzylamino)-thieno-[2,3-d]-pyrimidin;
mit 2,4-Dichlor-5,6-cyclohepteno-thieno-[2,3-d]-pyrimidin 2-Chlor-5,6-cyclohepteno-4-(4-fluorbenzylamino)-thieno-[2,3-d]-pyrimidin;
mit 2,4,5-Trichlor-6-methyl-thieno-[2,3-d]-pyrimidin 2,5-Dichlor-6-methyl-4-(4-fluorbenzylamino)-thieno-[2,3-d]-pyrimidin;
mit 2,4-Dichlor-5,6-dimethyl-thieno-[2,3-d]-pyrimidin 2-Chlor-5,6-dimethyl-4-(4-fluorbenzylamino)-thieno-[2,3-d]-pyrimidin.

Analog erhält man durch Umsetzung von 3,4-Dichlorbenzylamin
mit 2,4-Dichlor-5,6,7,8-tetrahydro-[1]-benzothieno-[2,3-d]-pyrimidin 2-Chlor-5,6,7,8-tetrahydro-4-(3,4-dichlorbenzylamino)-[1]-benzothieno-[2,3-d]-pyrimidin;
mit 2,4-Dichlor-5,6-cyclopenteno-thieno-[2,3-d]-pyrimidin 2-Chlor-5,6-cyclopenteno-4-(3,4-dichlorbenzylamino)-thieno-[2,3-d]-pyrimidin;
mit 2,4-Dichlor-5,6-cyclohepteno-thieno-[2,3-d]-pyrimidin 2-Chlor-5,6-cyclohepteno-4-(3,4-dichlorbenzylamino)-thieno-[2,3-d]-pyrimidin;
mit 2,4,5-Trichlor-6-methyl-thieno-[2,3-d]-pyrimidin 2,5-Dichlor-6-methyl-4-(3,4-dichlorbenzylamino)-thieno-[2,3-d]-pyrimidin;
mit 2,4-Dichlor-5,6-dimethyl-thieno-[2,3-d]-pyrimidin 2-Chlor-5,6-dimethyl-4-(3,4-dichlorbenzylamino)-thieno-[2,3-d]-pyrimidin.

Analog erhält man durch Umsetzung von 3,4-Ethylendioxybenzylamin
mit 2,4-Dichlor-5,6,7,8-tetrahydro-[1]-benzothieno-[2,3-d]-pyrimidin 2-Chlor-5,6,7,8-tetrahydro-4-(3,4-ethylendioxybenzylamino)-[1]-benzothieno-[2,3-d]-pyrimidin;
mit 2,4-Dichlor-5,6-cyclopenteno-thieno-[2,3-d]-pyrimidin 2-Chlor-5,6-cyclopenteno-4-(3,4-ethylendioxybenzylamino)-thieno-[2,3-d]-pyrimidin;
mit 2,4-Dichlor-5,6-cyclohepteno-thieno-[2,3-d]-pyrimidin 2-Chlor-5,6-cyclohepteno-4-(3,4-ethylendioxybenzylamino)-thieno-[2,3-d]-pyrimidin;
mit 2,4,5-Trichlor-6-methyl-thieno-[2,3-d]-pyrimidin 2,5-Dichlor-6-methyl-4-(3,4-ethylendioxybenzylamino)-thieno-[2,3-d]-pyrimidin;
mit 2,4-Dichlor-5,6-dimethyl-thieno-[2,3-d]-pyrimidin 2-Chlor-5,6-dimethyl-4-(3,4-ethylendioxybenzylamino)-thieno-[2,3-d]-pyrimidin.

### Beispiel 2

1,67 g 2-Chlor-6-methyl-4-(3,4-methylendioxybenzylamino)-thieno-[2,3-d]-pyrimidin, 1,02 g Imidazol und 2 g Phenol werden 5 Stunden bei 150 ° erhitzt. Nach Abkühlen wird der Rückstand in Dichlormethan gelöst und wie üblich aufgearbeitet. Man erhält 1,0 g 2-(lmidazol-1-yl)-6-methyl-4-(3,4-methylendioxybenzylamino)-thieno-[2,3-d]-pyrimidin, F. 248-250°.

Analog erhält man durch Umsetzung von Imidazol mit den unter Beispiel 1 erhaltenen 2-Chlor-thieno-[2,3-d]-pyrimidin-Derivaten, die in 4-Stellung Arylalkylamino-substituiert sind, die nachstehenden Verbindungen
2-(lmidazol-1-yl)-5,6,7,8-tetrahydro-4-(3,4-methylendioxybenzylamino)-[1]-benzothieno-[2,3-d]-pyrimidin, F. 218°;
2-(lmidazol-1-yl)-5,6-cyclopenteno-4-(3,4-methylendioxybenzylamino)-thieno-[2,3-d]-pyrimidin, F. 260°;
2-(lmidazol-1-yl)-5,6-cyclohepteno-4-(3,4-methylendioxybenzylamino)-thieno-[2,3-d]-pyrimidin, F. 210°;
2-(lmidazol-1-yl)-5-chlor-6-methyl-4-(3,4-methylendioxybenzylamino)-thieno-[2,3-d]-pyrimidin;
2-(lmidazol-1-yl)-5,6-dimethyl-4-(3,4-methylendioxybenzylamino)-thieno-[2,3-d]-pyrimidin, F. 245°
2-(Imidazol-1-yl)-5,6,7,8-tetrahydro-4-(3-chlor-4-methoxybenzylamino)-[1]-benzothieno-[2,3-d]-pyrimidin;
2-(lmidazol-1-yl)-5,6-cyclopenteno-4-(3-chlor-4-methoxybenzylamino)-thieno-[2,3-d]-pyrimidin;
2-(lmidazol-1-yl)-5,6-cyclohepteno-4-(3-chlor-4-methoxybenzylamino)-thieno-[2,3-d]-pyrimidin;
2-(Imidazol-1-yl)-5-chlor-6-methyl-4-(3-chlor-4-methoxybenzylamino)-thieno-[2,3-d]-pyrimidin;
2-(Imidazol-1-yl)-5,6-dimethyl-4-(3-chlor-4-methoxybenzylamino)-thieno-[2,3-d]-pyrimidin;
2-(lmidazol-1-yl)-5,6,7,8-tetrahydro-4-(3,4-dimethoxybenzylamino)-[1]-benzothieno-[2,3-d]-pyrimidin;
2-(lmidazol-1-yl)-5,6-cyclopenteno-4-(3,4-dimethoxybenzylamino)-thieno-[2,3-d]-pyrimidin;
2-(lmidazol-1-yl)-5,6-cyclohepteno-4-(3,4-dimethoxybenzylamino)-thieno-[2,3-d]-pyrimidin;
2-(Imidazol-1-yl)-5-chlor-6-methyl-4-(3,4-dimethoxybenzylamino)-thieno-[2,3-d]-pyrimidin;
2-(Imidazol-1-yl)-5,6-dimethyl-4-(3,4-dimethoxybenzylamino)-thieno-[2,3-d]-pyrimidin;
2-(Imidazol-1-yl)-5,6,7,8-tetrahydro-4-benzylamino-[1]-benzothieno-[2,3-d]-pyrimidin;
2-(lmidazol-1-yl)-5,6-cyclopenteno-4-benzylamino-thieno-[2,3-d]-pyrimidin;
2-(lmidazol-1-yl)-5,6-cyclohepteno-4-benzylamino-thieno-[2,3-d]-pyrimidin, F. 197°;
2-(lmidazol-1-yl)-5-chlor-6-methyl-4-benzylamino-thieno-[2,3-d]-pyrimidin;
2-(Imidazol-1-yl)-5,6,7,8-tetrahydro-4-(4-fluorbenzylamino)-[1]-benzothieno-[2,3-d]-pyrimidin;
2-(lmidazol-1-yl)-5,6-cyclopenteno-4-(4-fluorbenzylamino)-thieno-[2,3-d]-pyrimidin;
2-(Imidazol-1-yl)-5,6-cyclohepteno-4-(4-fluorbenzylamino)-thieno-[2,3-d]-pyrimidin;
2-(lmidazol-1-yl)-5-chlor-6-methyl-4-(4-fluorbenzylamino)-thieno-[2,3-d]-pyrimidin;
2-(Imidazol-1-yl)-5,6-dimethyl-4-(4-fluorbenzylamino)-thieno-[2,3-d]-pyrimidin;
2-(Imidazol-1-yl)-5,6,7,8-tetrahydro-4-(3,4-dichlorbenzylamino)-[1]-benzothieno-[2,3-d]-pyrimidin;
2-(Imidazol-1-yl)-5,6-cyclopenteno-4-(3,4-dichlorbenzylamino)-thieno-[2,3-d]-pyrimidin;
2-(Imidazol-1-yl)-5,6-cyclohepteno-4-(3,4-dichlorbenzylamino)-thieno-[2,3-d]-pyrimidin;
2-(Imidazol-1-yl)-5-chlor-6-methyl-4-(3,4-dichlorbenzylamino)-thieno-[2,3-d]-pyrimidin;
2-(lmidazol-1-yl)-5,6-dimethyl-4-(3,4-dichlorbenzylamino)-thieno-[2,3-d]-pyrimidin;
2-(Imidazol-1-yl)-5,6,7,8-tetrahydro-4-(3,4-ethylendioxybenzylamino)-[1]-benzothieno-[2,3-d]-pyrimidin;
2-(Imidazol-1-yl)-5,6-cyclopenteno-4-(3,4-ethylendioxybenzylamino)-thieno-[2,3-d]-pyrimidin;
2-(lmidazol-1 -yl)-5,6-cyclohepteno-4-(3,4-ethylendioxybenzylamino)-thieno-[2,3-d]-pyrimidin;
2-(Imidazol-1-yl)-5-chlor-6-methyl-4-(3,4-ethylendioxybenzylamino)-thieno-[2,3-d]-pyrimidin;
2-(lmidazol-1-yl)-5,6-dimethyl-4-(3,4-ethylendioxybenzylamino)-thieno-[2,3-d]-pyrimidin.

Analog erhält man durch Umsetzung von Pyrazol mit den unter Beispiel 1 erhaltenen 2-Chlor-thieno-[2,3-d]-pyrimidin-Derivaten, die in 4-Stellung Arylalkylamino-substituiert sind, die nachstehenden Verbindungen
2-(Pyrazol-1-yl)-5,6,7,8-tetrahydro-4-(3,4-methylendioxybenzylamino)-[1]-benzothieno-[2,3-d]-pyrimidin, F. 210°;
2-(Pyrazol-1-yl)-5,6-cyclopenteno-4-(3,4-methylendioxybenzylamino)-thieno-[2,3-d]-pyrimidin;
2-(Pyrazol-1-yl)-5,6-cyclohepteno-4-(3,4-methylendioxybenzylamino)-thieno-[2,3-d]-pyrimidin;
2-(Pyrazol-1-yl)-5-chlor-6-methyl-4-(3,4-methylendioxybenzylamino)-thieno-[2,3-d]-pyrimidin;
2-(Pyrazol-1-yl)-5,6-dimethyl-4-(3,4-methylendioxybenzylamino)-thieno-[2,3-d]-pyrimidin;
2-(Pyrazol-1-yl)-5,6,7,8-tetrahydro-4-(3-chlor-4-methoxybenzylamino)-[1]-benzothieno-[2,3-d]-pyrimidin;
2-(Pyrazol-1-yl)-5,6-cyclopenteno-4-(3-chlor-4-methyoxybenzylamino)-thieno-[2,3-d]-pyrimidin;
2-(Pyrazol-1-yl)-5,6-cyclohepteno-4-(3-chlor-4-methyoxybenzylamino)-thieno-[2,3-d]-pyrimidin;
2-(Pyrazol-1-yl)-5-chlor-6-methyl-4-(3-chlor-4-methoxybenzylamino)-thieno-[2,3-d]-pyrimidin;
2-(Pyrazol-1-yl)-5,6-dimethyl-4-(3-chlor-4-methoxybenzylamino)-thieno-[2,3-d]-pyrimidin;
2-(Pyrazol-1-yl)-5,6,7,8-tetrahydro-4-(3,4-dimethoxybenzylamino)-[1]-benzothieno-[2,3-d]-pyrimidin;
2-(Pyrazol-1-yl)-5,6-cyclopenteno-4-(3,4-dimethoxybenzylamino)-thieno-[2,3-d]-pyrimidin;
2-(Pyrazol-1-yl)-5,6-cyclohepteno-4-(3,4-dimethoxybenzylamino)-thieno-[2,3-d]-pyrimidin;
2-(Pyrazol-1-yl)-5-chlor-6-methyl-4-(3,4-dimethoxybenzylamino)-thieno-[2,3-d]-pyrimidin;
2-(Pyrazol-1-yl)-5,6-dimethyl-4-(3,4-dimethoxybenzylamino)-thieno-[2,3-d]-pyrimidin;
2-(Pyrazol-1-yl)-5,6,7,8-tetrahydro-4-benzylamino-[1]-benzothieno-[2,3-d]-pyrimidin;
2-(Pyrazol-1-yl)-5,6-cyclopenteno-4-benzylamino-thieno-[2,3-d]-pyrimidin;
2-(Pyrazol-1-yl)-5,6-cyclohepteno-4-benzylamino-thieno-[2,3-d]-pyrimidin;
2-(Pyrazol-1-yl)-5,6-dimethyl-4-benzylamino-thieno-[2,3-d]-pyrimidin;
2-(Pyrazol-1-yl)-5,6,7,8-tetrahydro-4-(4-fluorbenzylamino)-[1]-benzothieno-[2,3-d]-pyrimidin;
2-(Pyrazol-1-yl)-5,6-cyclopenteno-4-(4-fluorbenzylamino)-thieno-[2,3-d]-pyrimidin;
2-(Pyrazol-1-yl)-5,6-cyclohepteno-4-(4-fluorbenzylamino)-thieno-[2,3-d]-pyrimidin;
2-(Pyrazol-1-yl)-5-chlor-6-methyl-4-(4-fluorbenzylamino)-thieno-[2,3-d]-pyrimidin;
2-(Pyrazol-1-yl)-5,6-dimethyl-4-(4-fluorbenzylamino)-thieno-[2,3-d]-pyrimidin;
2-(Pyrazol-1-yl)-5,6,7,8-tetrahydro-4-(3,4-dichlorbenzylamino)-[1]-benzothieno-[2,3-d]-pyrimidin;
2-(Pyrazol-1 -yl)-5,6-cyclopenteno-4-(3,4-dichlorbenzylamino)-thieno-[2,3-d]-pyrimidin;
2-(Pyrazol-1-yl)-5,6-cyclohepteno-4-(3,4-dichlorbenzylamino)-thieno-[2,3-d]-pyrimidin;
2-(Pyrazol-1-yl)-5-chlor-6-methyl-4-(3,4-dichlorbenzylamino)-thieno-[2,3-d]-pyrimidin;
2-(Pyrazol-1-yl)-5,6-dimethyl-4-(3,4-dichlorbenzylamino)-thieno-[2,3-d]-pyrimidin;
2-(Pyrazol-1-yl)-5,6,7,8-tetrahydro-4-(3,4-ethylendioxybenzylamino)-[1]-benzothieno-[2,3-d]-pyrimidin;
2-(Pyrazol-1-yl)-5,6-cyclopenteno-4-(3,4-ethylendioxybenzylamino)-thieno-[2,3-d]-pyrimidin;
2-(Pyrazol-1-yl)-5,6-cyclohepteno-4-(3,4-ethylendioxybenzylamino)-thieno-[2,3-d]-pyrimidin;
2-(Pyrazol-1 -yl)-5-chlor-6-methyl-4-(3,4-ethylendioxybenzylamino)-thieno-[2,3-d]-pyrimidin;
2-(Pyrazol-1-yl)-5,6-dimethyl-4-(3,4-ethylendioxybenzylamino)-thieno-[2,3-d]-pyrimidin.

Analog erhält man durch Umsetzung von 1,2,4-Triazol mit den unter Beispiel 1 erhaltenen 2-Chlor-thieno-[2,3-d]-pyrimidin-Derivaten, die in 4-Stellung Arylalkylamino-substituiert sind, die nachstehenden Verbindungen
2-(1,2,4-Triazol-1-yl)-5,6,7,8-tetrahydro-4-(3,4-methylendioxybenzylamino)-[1]-benzothieno-[2,3-d]-pyrimidin;
2-(1,2,4-Triazol-1-yl)-5,6-cyclopenteno-4-(3,4-methylendioxybenzylamino)-thieno-[2,3-d]-pyrimidin;
2-(1,2,4-Triazol-1-yl)-5,6-cyclohepteno-4-(3,4-methylendioxybenzylamino)-thieno-[2,3-d]-pyrimidin;
2-(1 ,2,4-Triazol-1 -yl)-5-chlor-6-methyl-4-(3,4-methylendioxybenzylamino)-thieno-[2,3-d]-pyrimidin;
2-(1,2,4-Triazol-1-yl)-5,6-dimethyl-4-(3,4-methylendioxybenzylamino)-thieno-[2,3-d]-pyrimidin;
2-(1,2,4-Triazol-1-yl)-5,6,7,8-tetrahydro-4-(3-chlor-4-methoxybenzylamino)-[1]-benzothieno-[2,3-d]-pyrimidin;
2-(1 ,2,4-Triazol-1 -yl)-5,6-cyclopenteno-4-(3-chlor-4-methyoxybenzylamino)-thieno-[2,3-d]-pyrimidin;
2-(1,2,4-Triazol-1-yl)-5,6-cyclohepteno-4-(3-chlor-4-methyoxybenzylamino)-thieno-[2,3-d]-pyrimidin;
2-(1,2,4-Triazol-1-yl)-5-chlor-6-methyl-4-(3-chlor-4-methoxybenzylamino)-thieno-[2,3-d]-pyrimidin;
2-(1,2,4-Triazol-1-yl)-5,6-dimethyl-4-(3-chlor-4-methoxybenzylamino)-thieno-[2,3-d]-pyrimidin;
2-(1,2,4-Triazol-1-yl)-5,6,7,8-tetrahydro-4-(3,4-dimethoxybenzylamino)-[1]-benzothieno-[2,3-d]-pyrimidin;
2-(1,2,4-Triazol-1-yl)-5,6-cyclopenteno-4-(3,4-dimethoxybenzylamino)-thieno-[2,3-d]-pyrimidin;
2-(1,2,4-Triazol-1-yl)-5,6-cyclohepteno-4-(3,4-dimethoxybenzylamino)-thieno-[2,3-d]-pyrimidin;
2-(1,2,4-Triazol-1-yl)-5-chlor-6-methyl-4-(3,4-dimethoxybenzylamino)-thieno-[2,3-d]-pyrimidin;
2-(1,2,4-Triazol-1-yl)-5,6-dimethyl-4-(3,4-dimethoxybenzylamino)-thieno-[2,3-d]-pyrimidin;
2-(1,2,4-Triazol-1-yl)-5,6,7,8-tetrahydro-4-benzylamino-[1]-benzothieno-[2,3-d]-pyrimidin;
2-(1,2,4-Triazol-1-yl)-5,6-cyclopenteno-4-benzylamino-thieno-[2,3-d]-pyrimidin;
2-(1,2,4-Triazol-1-yl)-5,6-cyclohepteno-4-benzylamino-thieno-[2,3-d]-pyrimidin;
2-(1,2,4-Triazol-1-yl)-5,6,7,8-tetrahydro-4-(4-fluorbenzylamino)-[1]-benzothieno-[2,3-d]-pyrimidin;
2-(1,2,4-Triazol-1-yl)-5,6-cyclopenteno-4-(4-fluorbenzylamino)-thieno-[2,3-d]-pyrimidin;
2-(1,2,4-Triazol-1-yl)-5,6-cyclohepteno-4-(4-fluorbenzylamino)-thieno-[2,3-d]-pyrimidin;
2-(1,2,4-Triazol-1-yl)-5-chlor-6-methyl-4-(4-fluorbenzylamino)-thieno-[2,3-d]-pyrimidin;
2-(1,2,4-Triazol-1-yl)-5,6-dimethyl-4-(4-fluorbenzylamino)-thieno-[2,3-d]-pyrimidin;
2-(1,2,4-Triazol-1-yl)-5,6,7,8-tetrahydro-4-(3,4-dichlorbenzylamino)-[1]-benzothieno-[2,3-d]-pyrimidin;
2-(1,2,4-Triazol-1-yl)-5,6-cyclopenteno-4-(3,4-dichlorbenzylamino)-thieno-[2,3-d]-pyrimidin;
2-(1,2,4-Triazol-1-yl)-5,6-cyclohepteno-4-(3,4-dichlorbenzylamino)-thieno-[2,3-d]-pyrimidin;
2-(1,2,4-Triazol-1-yl)-5-chlor-6-methyl-4-(3,4-dichlorbenzylamino)-thieno-[2,3-d]-pyrimidin;
2-(1,2,4-Triazol-1-yl)-5,6-dimethyl-4-(3,4-dichlorbenzylamino)-thieno-[2,3-d]-pyrimidin;
2-(1,2,4-Triazol-1-yl)-5,6,7,8-tetrahydro-4-(3,4-ethylendioxybenzylamino)-[1]-benzothieno-[2,3-d]-pyrimidin;
2-(1,2,4-Triazol-1-yl)-5,6-cyclopenteno-4-(3,4-ethylendioxybenzylamino)-thieno-[2,3-d]-pyrimidin;
2-(1,2,4-Triazol-1-yl)-5,6-cyclohepteno-4-(3,4-ethylendioxybenzylamino)-thieno-[2,3-d]-pyrimidin;
2-(1,2,4-Triazol-1-yl)-5-chlor-6-methyl-4-(3,4-ethylendioxybenzylamino)-thieno-[2,3-d]-pyrimidin;
2-(1,2,4-Triazol-1-yl)-5,6-dimethyl-4-(3,4-ethylendioxybenzylamino)-thieno-[2,3-d]-pyrimidin.

Analog erhält man durch Umsetzung von 2-Methylimidazol mit den unter Beispiel 1 erhaltenen 2-Chlor-thieno-[2,3-d]-pyrimidin-Derivaten, die in 4-Stellung Arylalkylamino-substituiert sind, die nachstehenden Verbindungen
2-(2-Methylimidazol-1-yl)-5,6,7,8-tetrahydro-4-(3,4-methylendioxybenzylamino)-[1]-benzothieno-[2,3-d]-pyrimidin, amorph;
2-(2-Methylimidazol-1-yl)-5,6-cyclopenteno-4-(3,4-methylendioxybenzylamino)-thieno-[2,3-d]-pyrimidin;
2-(2-Methylimidazol-1-yl)-5,6-cyclohepteno-4-(3,4-methylendioxybenzylamino)-thieno-[2,3-d]-pyrimidin;
2-(2-Methylimidazol-1-yl)-5-chlor-6-methyl-4-(3,4-methylendioxybenzylamino)-thieno-[2,3-d]-pyrimidin;
2-(2-Methylimidazol-1-yl)-5,6-dimethyl-4-(3,4-methylendioxybenzylamino)-thieno-[2,3-d]-pyrimidin;
2-(2-Methylimidazol-1-yl)-5,6,7,8-tetrahydro-4-(3-chlor-4-methoxybenzylamino)-[1]-benzothieno-[2,3-d]-pyrimidin;
2-(2-Methylimidazol-1-yl)-5,6-cyclopenteno-4-(3-chlor-4-methyoxybenzylamino)-thieno-[2,3-d]-pyrimidin;
2-(1,2,4-Triazol-1-yl)-5,6-cyclohepteno-4-(3-chlor-4-methoxybenzylamino)-thieno-[2,3-d]-pyrimidin;
2-(2-Methylimidazol-1-yl)-5-chlor-6-methyl-4-(3-chlor-4-methoxybenzylamino)-thieno-[2,3-d]-pyrimidin;
2-(2-Methylimidazol-1-yl)-5,6-dimethyl-4-(3-chlor-4-methoxybenzylamino)-thieno-[2,3-d]-pyrimidin;
2-(2-Methylimidazol-1-yl)-5,6,7,8-tetrahydro-4-(3,4-dimethoxybenzylamino)-[1]-benzothieno-[2,3-d]-pyrimidin;
2-(2-Methylimidazol-1-yl)-5,6-cyclopenteno-4-(3,4-dimethoxybenzylamino)-thieno-[2,3-d]-pyrimidin;
2-(2-Methylimidazol-1-yl)-5,6-cyclohepteno-4-(3,4-dimethoxybenzylamino)-thieno-[2,3-d]-pyrimidin;
2-(2-Methylimidazol-1-yl)-5-chlor-6-methyl-4-(3,4-dimethoxybenzylamino)-thieno-[2,3-d]-pyrimidin;
2-(2-Methylimidazol-1-yl)-5,6-dimethyl-4-(3,4-dimethoxybenzylamino)-thieno-[2,3-d]-pyrimidin;
2-(2-Methylimidazol-1-yl)-5,6,7,8-tetrahydro-4-benzylamino-[1]-benzothieno-[2,3-d]-pyrimidin;
2-(2-Methylimidazol-1-yl)-5,6-cyclopenteno-4-benzylamino-thieno-[2,3-d]-pyrimidin;
2-(2-Methylimidazol-1-yl)-5,6-cyclohepteno-4-benzylamino-thieno-[2,3-d]-pyrimidin;
2-(2-Methylimidazol-1-yl)-5,6,7,8-tetrahydro-4-(4-fluorbenzylamino)-[1]-benzothieno-[2,3-d]-pyrimidin;
2-(2-Methylimidazol-1-yl)-5,6-cyclopenteno-4-(4-fluorbenzylamino)-thieno-[2,3-d]-pyrimidin;
2-(2-Methylimidazol-1-yl)-5,6-cyclohepteno-4-(4-fluorbenzylamino)-thieno-[2,3-d]-pyrimidin;
2-(2-Methylimidazol-1-yl)-5-chlor-6-methyl-4-(4-fluorbenzylamino)-thieno-[2, 3-d]-pyrimidin;
2-(2-Methylimidazol-1-yl)-5,6-dimethyl-4-(4-fluorbenzylamino)-thieno-[2,3-d]-pyrimidin;
2-(2-Methylimidazol-1-yl)-5,6,7,8-tetrahydro-4-(3,4-dichlorbenzylamino)-[1]-benzothieno-[2,3-d]-pyrimidin;
2-(2-Methylimidazol-1-yl)-5,6-cyclopenteno-4-(3,4-dichlorbenzylamino)-thieno-[2,3-d]-pyrimidin;
2-(2-Methylimidazol-1-yl)-5,6-cyclohepteno-4-(3,4-dichlorbenzylamino)-thieno-[2,3-d]-pyrimidin;
2-(2-Methylimidazol-1-yl)-5-chlor-6-methyl-4-(3,4-dichlorbenzylamino)-thieno-[2,3-d]-pyrimidin;
2-(2-Methylimidazol-1-yl)-5,6-dimethyl-4-(3,4-dichlorbenzylamino)-thieno-[2,3-d]-pyrimidin;
2-(2-Methylimidazol-1-yl)-5,6,7,8-tetrahydro-4-(3,4-ethylendioxybenzylamino)-[1]-benzothieno-[2,3-d]-pyrimidin;
2-(2-Methylimidazol-1-yl)-5,6-cyclopenteno-4-(3,4-ethylendioxybenzylamino)-thieno-[2,3-d]-pyrimidin;
2-(2-Methylimidazol-1-yl)-5,6-cyclohepteno-4-(3,4-ethylendioxybenzylamino)-thieno-[2,3-d]-pyrimidin;
2-(2-Methylimidazol-1-yl)-5-chlor-6-methyl-4-(3,4-ethylendioxybenzylamino)-thieno-[2,3-d]-pyrimidin;
2-(2-Methylimidazol-1-yl)-5,6-dimethyl-4-(3,4-ethylendioxybenzylamino)-thieno-[2,3-d]-pyrimidin.

### Beispiel 3

5 g 2-Amino-5-methyl-3-ethoxycarbonyl-thiophen wird mit 2,7 g 3-Cyanpyridin in 40 ml Dioxan gelöst. Anschließend wird für 5 Stunden gasförmiges HCI durch die Lösung geleitet. Nach üblicher Aufarbeitung erhält man 6 g 3,4-Dihydro-4-oxo-2-(pyridin-3-yl)-6-methyl-thieno-[2,3-d]-pyrimidin.
Der Ersatz der Ketogruppe durch Cl unter Ausbildung des aromatischen Pyrimidinrings erfolgt unter Standardbedingungen.
Eine Mischung aus 18 ml POCl₃ mit 6 g 3,4-Dihydro-4-oxo-2-(pyridin-3-yl)-6-methyl-thieno-[2,3-d]-pyrimidin unter Zusatz von 1,8 ml N,N-Dimethylanilin wird 4 Stunden gekocht. Nach üblicher Aufarbeitung erhält man 5 g 4-Chlor-2-(pyridin-3-yl)-6-methyl-thieno-[2,3-d]-pyrimidin.

Analog erhält man durch Umsetzung von 3-Cyanpyridin und anschließender Reaktion mit POCl₃
aus 2-Amino-4,5,6,7-tetrahydro-3-ethoxycarbonyl-benzothiophen 4-Chlor-2-(pyridin-3-yl)-5,6,7,8-tetrahydro-[1]-benzothieno-[2,3-d]-pyrimidin, F. 143°;
aus 2-Amino-4,5-cyclopenteno-3-ethoxycarbonyl-thiophen 4-Chlor-2-(pyridin-3-yl)-5,6-cyclopenteno-thieno-[2,3-d]-pyrimidin;
aus 2-Amino-4,5-cyclohepteno-3-ethoxycarbonyl-thiophen 4-Chlor-2-(pyridin-3-yl)-5,6-cyclohepteno-thieno-[2,3-d]-pyrimidin;
aus 2-Amino-4-chlor-5-methyl-3-ethoxycarbonyl-thiophen 4-Chlor-2-(pyridin-3-yl)-5-chlor-6-methyl-thieno-[2,3-d]-pyrimidin;
aus 2-Amino-4,5-dimethyl-3-ethoxycarbonyl-thiophen 4-Chlor-2-(pyridin-3-yl)-5,6-dimethyl-thieno-[2,3-d]-pyrimidin;

### Beispiel 4

Analog Beispiel 1 erhält man durch Umsetzung von 3,4-Methylendioxybenzylamin
mit 4-Chlor-2-(pyridin-3-yl)-5,6,7,8-tetrahydro-[1]-benzothieno-[2,3-d]-pyrimidin 2-(Pyridin-3-yl)-4-(3,4-methylendioxybenzylamino)-5,6,7,8-tetrahydro-[1]-benzothieno-[2,3-d]-pyrimidin, F. 215°;
mit 4-Chlor-2-(pyridin-3-yl)-5,6-cyclopenteno-thieno-[2,3-d]-pyrimidin 2-(Pyridin-3-yl)-4-(3,4-methylendioxybenzylamino)-5,6-cyclopentenothieno-[2,3-d]-pyrimidin;
mit 4-Chlor-2-(pyridin-3-yl)-5,6-cyclohepteno-thieno-[2,3-d]-pyrimidin 2-(Pyridin-3-yl)-4-(3,4-methylendioxybenzylamino)-5,6-cycloheptenothieno-[2,3-d]-pyrimidin;
mit 4-Chlor-2-(pyridin-3-yl)-5-Chlor-6-methyl-thieno-[2,3-d]-pyrimidin 2-(Pyridin-3-yl)-4-(3,4-methylendioxybenzylamino)-5-chlor-6-methylthieno-[2,3-d]-pyrimidin;
mit 4-Chlor-2-(pyridin-3-yl)-5,6-dimethyl-thieno-[2,3-d]-pyrimidin 2-(Pyridin-3-yl)-4-(3,4-methylendioxybenzylamino)-5,6-dimethylthieno-[2,3-d]-pyrimidin.

Analog erhält man durch Umsetzung von 3-Chlor-4-methoxy-benzylamin
mit 4-Chlor-2-(pyridin-3-yl)-5,6,7,8-tetrahydro-[1]-benzothieno-[2,3-d]-pyrimidin 2-(Pyridin-3-yl)-4-(3-chlor-4-methoxybenzylamino)-5,6,7,8-tetrahydro-[1]-benzothieno-[2,3-d]-pyrimidin;
mit 4-Chlor-2-(pyridin-3-yl)-5,6-cyclopenteno-thieno-[2,3-d]-pyrimidin 2-(Pyridin-3-yl)-4-(3-chlor-4-methoxybenzylamino)-5,6-cyclopentenothieno-[2,3-d]-pyrimidin;
mit 4-Chlor-2-(pyridin-3-yl)-5,6-cyclohepteno-thieno-[2,3-d]-pyrimidin 2-(Pyridin-3-yl)-4-(3-chlor-4-methoxybenzylamino)-5,6-cycloheptenothieno-[2,3-d]-pyrimidin;
mit 4-Chlor-2-(pyridin-3-yl)-5-chlor-6-methyl-thieno-[2,3-d]-pyrimidin 2-(Pyridin-3-yl)-4-(3-chlor-4-methoxybenzylamino)-5-chlor-6-methylthieno-[2,3-d]-pyrimidin;
mit 4-Chlor-2-(pyridin-3-yl)-5,6-dimethyl-thieno-[2,3-d]-pyrimidin 2-(Pyridin-3-yl)-4-(3-chlor-4-methoxybenzylamino)-5,6-dimethylthieno-[2,3-d]-pyrimidin.

Analog erhält man durch Umsetzung von 3,4-Dimethoxy-benzylamin
mit 4-Chlor-2-(pyridin-3-yl)-5,6,7,8-tetrahydro-[1]-benzothieno-[2,3-d]-pyrimidin 2-(Pyridin-3-yl)-4-(3,4-dimethoxybenzylamino)-5,6,7,8-tetra-hydro-[1]-benzothieno-[2,3-d]-pyrimidin;
mit 4-Chlor-2-(pyridin-3-yl)-5,6-cyclopenteno-thieno-[2,3-d]-pyrimidin 2-(Pyridin-3-yl)-4-(3,4-dimethoxybenzylamino)-5,6-cyclopentenothieno-[2,3-d]-pyrimidin;
mit 4-Chlor-2-(pyridin-3-yl)-5,6-cyclohepteno-thieno-[2,3-d]-pyrimidin 2-(Pyridin-3-yl)-4-(3,4-dimethoxybenzylamino)-5,6-cycloheptenothieno-[2,3-d]-pyrimidin;
mit 4-Chlor-2-(pyridin-3-yl)-5-chlor-6-methyl-thieno-[2,3-d]-pyrimidin 2-(Pyridin-3-yl)-4-(3,4-dimethoxybenzylamino)-5-chlor-6-methylthieno-[2,3-d]-pyrimidin;
mit 4-Chlor-2-(pyridin-3-yl)-5,6-dimethyl-thieno-[2,3-d]-pyrimidin 2-(Pyridin-3-yl)-4-(3,4-dimethoxybenzylamino)-5,6-dimethyl-thieno-[2,3-d]-pyrimidin.

Analog erhält man durch Umsetzung von Benzylamin
mit 4-Chlor-2-(pyridin-3-yl)-5,6,7,8-tetrahydro-[1]-benzothieno-[2,3-d]-pyrimidin 2-(Pyridin-3-yl)-4-benzylamino-5,6,7,8-tetrahydro-[1]-benzothieno-[2,3-d]-pyrimidin, F. 189°;
mit 4-Chlor-2-(pyridin-3-yl)-5,6-cyclopenteno-thieno-[2,3-d]-pyrimidin 2-(Pyridin-3-yl)-4-benzylamino-5,6-cyclopenteno-thieno-[2,3-d]-pyrimidin;
mit 4-Chlor-2-(pyridin-3-yl)-5,6-cyclohepteno-thieno-[2,3-d]-pyrimidin 2-(Pyridin-3-yl)-4-benzylamino-5,6-cyclohepteno-thieno-[2,3-d]-pyrimidin.

Analog erhält man durch Umsetzung von 4-Fluorbenzylamin
mit 4-Chlor-2-(pyridin-3-yl)-5,6,7,8-tetrahydro-[1]-benzothieno-[2,3-d]-pyrimidin 2-(Pyridin-3-yl)-4-(4-fluorbenzylamino)-5,6,7,8-tetrahydro-[1]-benzothieno-[2,3-d]-pyrimidin;
mit 4-Chlor-2-(pyridin-3-yl)-5,6-cyclopenteno-thieno-[2,3-d]-pyrimidin 2-(Pyridin-3-yl)-4-(4-fluorbenzylamino)-5,6-cyclopenteno-thieno-[2,3-d]-pyrimidin;
mit 4-Chlor-2-(pyridin-3-yl)-5,6-cyclohepteno-thieno-[2,3-d]-pyrimidin 2-(Pyridin-3-yl)-4-(4-fluorbenzylamino)-5,6-cyclohepteno-thieno-[2,3-d]-pyrimidin;
mit 4-Chlor-2-(pyridin-3-yl)-5-chlor-6-methyl-thieno-[2,3-d]-pyrimidin 2-(Pyridin-3-yl)-4-(4-fluorbenzylamino)-5-chlor-6-methyl-thieno-[2,3-d]-pyrimidin;
mit 4-Chlor-2-(pyridin-3-yl)-5,6-dimethyl-thieno-[2,3-d]-pyrimidin 2-(Pyridin-3-yl)-4-(4-fluorbenzylamino)-5,6-dimethyl-thieno-[2,3-d]-pyrimidin.

Analog erhält man durch Umsetzung von 3,4-Dichlorbenzylamin
mit 4-Chlor-2-(pyridin-3-yl)-5,6,7,8-tetrahydro-[1]-benzothieno-[2,3-d]-pyrimidin 2-(Pyridin-3-yl)-4-(3,4-dichlorbenzylamino)-5,6,7,8-tetrahydro-[1]-benzothieno-[2,3-d]-pyrimidin;
mit 4-Chlor-2-(pyridin-3-yl)-5,6-cyclopenteno-thieno-[2,3-d]-pyrimidin 2-(Pyridin-3-yl)-4-(3,4-dichlorbenzylamino)-5,6-cyclopenteno-thieno-[2,3-d]-pyrimidin;
mit 4-Chlor-2-(pyridin-3-yl)-5,6-cyclohepteno-thieno-[2,3-d]-pyrimidin 2-(Pyridin-3-yl)-4-(3,4-dichlorbenzylamino)-5,6-cyclohepteno-thieno-[2,3-d]-pyrimidin;
mit 4-Chlor-2-(pyridin-3-yl)-5-chlor-6-methyl-thieno-[2,3-d]-pyrimidin 2-(Pyridin-3-yl)-4-(3,4-dichlorbenzylamino)-5-chlor-6-methyl-thieno-[2,3-d]-pyrimidin;
mit 4-Chlor-2-(pyridin-3-yl)-5,6-dimethyl-thieno-[2,3-d]-pyrimidin 2-(Pyridin-3-yl)-4-(3,4-dichlorbenzylamino)-5,6-dimethyl-thieno-[2,3-d]-pyrimidin.

Analog erhält man durch Umsetzung von 3,4-Ethylendioxybenzylamin
mit 4-Chlor-2-(pyridin-3-yl)-5,6,7,8-tetrahydro-[1]-benzothieno-[2,3-d]-pyrimidin 2-(Pyridin-3-yl)-4-(3,4-ethylendioxybenzylamino)-5,6,7,8-tetrahydro-[1]-benzothieno-[2,3-d]-pyrimidin;
mit 4-Chlor-2-(pyridin-3-yl)-5,6-cyclopenteno-thieno-[2,3-d]-pyrimidin 2-(Pyridin-3-yl)-4-(3,4-ethylendioxybenzylamino)-5,6-cyclopentenothieno-[2,3-d]-pyrimidin;
mit 4-Chlor-2-(pyridin-3-yl)-5,6-cyclohepteno-thieno-[2,3-d]-pyrimidin 2-(Pyridin-3-yl)-4-(3,4-ethylendioxybenzylamino)-5,6-cycloheptenothieno-[2,3-d]-pyrimidin;
mit 4-Chlor-2-(pyridin-3-yl)-5-chlor-6-methyl-thieno-[2,3-d]-pyrimidin 2-(Pyridin-3-yl)-4-(3,4-ethylendioxybenzylamino)-5-chlor-6-methylthieno-[2,3-d]-pyrimidin;
mit 4-Chlor-2-(pyridin-3-yl)-5,6-dimethyl-thieno-[2,3-d]-pyrimidin 2-(Pyridin-3-yl)-4-(3,4-ethylendioxybenzylamino)-5,6-dimethyl-thieno-[2,3-d]-pyrimidin.

### Beispiel 5

Analog Beispiel 2 erhält man die nachstehenden Verbindungen
2-(lmidazol-1-yl)-5,6,7,8-tetrahydro-4-(3,4-difluorbenzylamino)-[1]-benzothieno-[2,3-d]-pyrimidin, F. 212°;
2-(Imidazol-1-yl)-5,6-cyclopenteno-4-benzylamino-thieno-[2,3-d]-pyrimidin, F. 221°;
2-(lmidazol-1-yl)-6-chlor-5-methyl-4-(3,4-methylendioxybenzylamino)-thieno-[2,3-d]-pyrimidin, F. 250°;
2-(Imidazol-1-yl)-5,6,7,8-tetrahydro-4-benzylamino--[1]-benzothieno-[2,3-d]-pyrimidin, F. 190°.

Die nachfolgenden Beispiele betreffen pharmazeutische Zubereitungen:

### Beispiel A: Injektionsgläser

Eine Lösung von 100 g eines Wirkstoffes der Formel I und 5 g Dinatriumhydrogenphosphat wird in 3 l zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I, 9,38 g NaH₂PO₄ · 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 I auf und sterilisiert durch Bestrahlung. Diese Lösung kann in Form von Augentropfen verwendet werden.

### Beispiel D: Salbe

Man mischt 500 mg eines Wirkstoffes der Formel I mit 99,5 g Vaseline unter aseptischen Bedingungen.

### Beispiel E: Tabletten

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I in 60 I zweifach destilliertem Wasser wird steril filtriert, in Ampullen abgefüllt, unter sterilen Bedingungen lyophilisiert und steril verschlossen. Jede Ampulle enthält 10 mg Wirkstoff.

### Beispiel I: Inhalations-Spray

Man löst 14 g Wirkstoff der Formel I in 10 I isotonischer NaCI-Lösung und füllt die Lösung in handelsübliche Sprühgefäße mit Pump-Mechanismus. Die Lösung kann in Mund oder Nase gesprüht werden. Ein Sprühstoß (etwa 0,1 ml) entspricht einer Dosis von etwa 0,14 mg.

## Patentansprüche

1. Verbindungen der Formel I 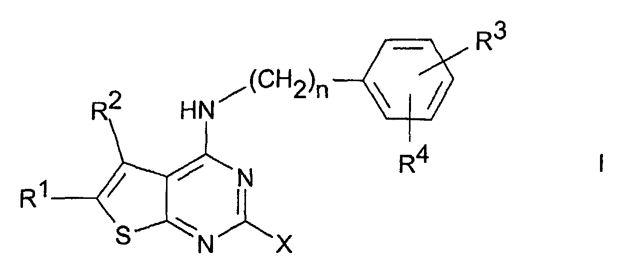 worin
R¹, R² jeweils unabhängig voneinander A oder Hal,
R¹ und R² zusammen auch Alkylen mit 3-5 C-Atomen,
R³, R⁴ jeweils unabhängig voneinander H, OA oder Hal,
R³ und R⁴ zusammen auch -O-CH₂-O- oder -O-CH₂-CH₂-O-,
A Alkyl mit 1 bis 6 C-Atomen,
X 1-Imidazolyl, 2-Methyl-imidazolyl, Pyrazolyl, 1,2,4-Triazol-1-yl oder 3-Pyridyl,
Hal F, Cl, Br oder I
und
n 1
bedeuten,
mit der Maßgabe, daß wenn
R¹, R² jeweils unabhängig voneinander A oder Hal,
bedeuten,
dann R³ und R⁴ nicht H bedeuten,
sowie deren physiologisch unbedenklichen Salze.

2. Verbindungen der Formel I gemäß Anspruch 1
(a) 2-(1-Imidazolyl)-5,6-dimethyl-4-(3,4-methylendioxy-benzyl-amino)-thieno-[2,3-d]-pyrimidin;
(b) 2-(1-Imidazolyl)-4-(3,4-methylendioxy-benzylamino)-5,6,7,8-tetrahydro-[1]-benzothieno-[2,3-d]-pyrimidin;
(c) 2-(1,2,4-Triazol-1-yl)-4-(3,4-methylendioxy-benzylamino)-5,6,7,8-tetrahydro-[1]-benzothieno-[2,3-d]-pyrimidin;
(d) 2-(Pyrazol-1-yl)-4-(3,4-methylendioxy-benzylamino)-5,6,7,8-tetrahydro-[1]-benzothieno-[2,3-d]-pyrimidin;
(e) 2-(Pyridin-3-yl)-4-(3,4-methylendioxy-benzylamino)-5,6,7,8-tetrahydro-[1]-benzothieno-[2,3-d]-pyrimidin.

3. Verfahren zur Herstellung
von Verbindungen der Formel I nach Anspruch 1 sowie deren Salzen, worin X über C gebunden ist,
**dadurch gekennzeichnet, daß** man eine Verbindung der Formel III 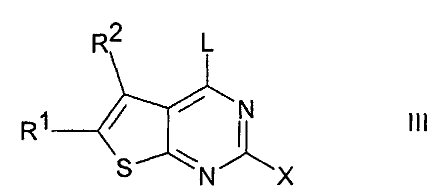 worin
R¹, R² und X die angegebenen Bedeutungen haben,
und L Cl, Br, OH, SCH₃ oder eine reaktionsfähige veresterte OH-Gruppe bedeutet,
mit einer Verbindung der Formel IV 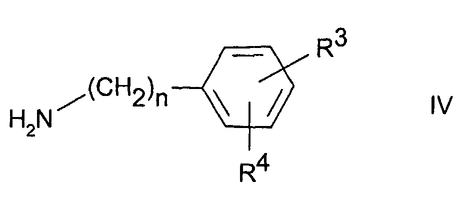 worin
R³, R⁴ und n die angegebenen Bedeutungen haben,
umsetzt,
und/oder daß man eine basische Verbindung der Formel I durch Behandeln mit einer Säure in eines ihrer Salze überführt.

4. Verfahren zur Herstellung pharmazeutischer Zubereitungen, **dadurch gekennzeichnet, daß** man eine Verbindung der Formel I nach Anspruch 1 und/oder eines ihrer physiologischen unbedenklichen Salze zusammen mit mindestens einem festen, flüssigen oder halbflüssigen Träger- oder Hilfsstoff in eine geeignete Dosierungsform bringt.

5. Pharmazeutische Zubereitung, **gekennzeichnet durch** einen Gehalt an mindestens einer Verbindung der Formel I nach Anspruch 1 und/oder einem ihrer physiologisch unbedenklichen Salze.

6. Verbindungen der Formel I nach Anspruch 1 und ihre physiologisch unbedenklichen Salze zur Bekämpfung von Krankheiten des Herz-Kreislaufsystems und zur Therapie von Potenzstörungen .

7. Arzneimittel der Formel I nach Anspruch 1 und ihre physiologisch unbedenklichen Salze als Phosphodiesterase V-Hemmer.

8. Verwendung von Verbindungen der Formel I nach Anspruch 1 und/oder ihre physiologisch unbedenklichen Salze zur Herstellung eines Arzneimittels.

## Claims

1. Compounds of the formula I 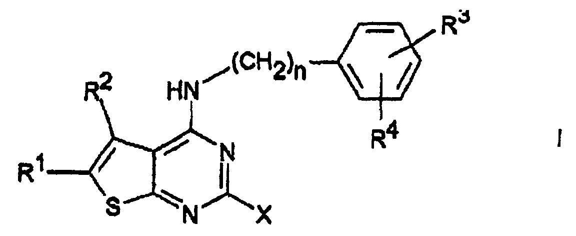 in which
R¹ and R² are each, independently of one another, A or Hal,
R¹ and R² together are alternatively alkylene having 3-5 carbon atoms,
R³ and R⁴ are each, independently of one another, H, OA or Hal,
R³ and R⁴ together are alternatively -O-CH₂-O- or -O-CH₂-CH₂-O-,
A is alkyl having from 1 to 6 carbon atoms,
X is 1-imidazolyl, 2-methylimidazolyl, pyrazolyl, 1,2,4-triazol-1-yl or 3-pyridyl,
Hal is F, Cl, Br or I,
and
n is 1,
with the proviso that if
R¹ and R² are each, independently of one another, A or Hal,
then R³ and R⁴ are not H,
and physiologically acceptable salts thereof.

2. Compounds of the formula I according to Claim 1
(a) 2-(1-imidazolyl)-5,6-dimethyl-4-(3,4-methylenedioxybenzylamino)thieno-[2,3-d]-pyrimidine;
(b) 2-(1-imidazolyl)-4-(3,4-methylenedioxybenzylamino)-5,6,7,8-tetrahydro-[1]-benzothieno-[2,3-d]-pyrimidine;
(c) 2-(1,2,4-triazol-1-yl)-4-(3,4-methylenedioxybenzylamino)-5,6,7,8-tetrahydro-[1]-benzothieno-[2,3-d]-pyrimidine;
(d) 2-(pyrazol-1-yl)-4-(3,4-methylenedioxybenzylamino)-5,6,7,8-tetrahydro-[1]-benzothieno-[2,3-d]-pyrimidine;
(e) 2-(pyridin-3-yl)-4-(3,4-methylenedioxybenzylamino)-5,6,7,8-tetrahydro-[1]-benzothieno-[2,3-d]-pyrimidine.

3. Process for the preparation
of compounds of the formula I according to Claim 1 and salts thereof in which X is bonded via C,
**characterised in that** a compound of the formula III 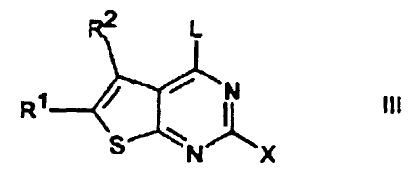 in which
R¹, R² and X have the meanings indicated,
and L is Cl, Br, OH, SCH₃ or a reactive esterified OH group,
is reacted with a compound of the formula IV 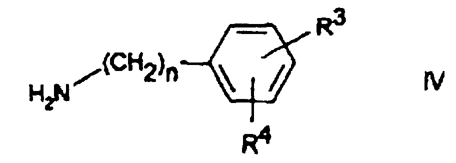 in which
R³, R⁴ and n have the meanings indicated,
and/or **in that** a basic compound of the formula I is converted into one of its salts by treatment with an acid.

4. Process for the preparation of pharmaceutical preparations, **characterised in that** a compound of the formula I according to Claim 1 and/or a physiologically acceptable salt thereof is converted into a suitable dosage form together with at least one solid, liquid or semiliquid excipient or adjuvant.

5. Pharmaceutical preparation, **characterised by** a content of at least one compound of the formula I according to Claim 1 and/or a physiologically acceptable salt thereof.

6. Compounds of the formula I according to Claim 1 and physiologically acceptable salts thereof for combating diseases of the cardiovascular system and for the therapy of potency disorders.

7. Medicament of the formula I according to Claim 1 and physiologically acceptable salts thereof as phosphodiesterase V inhibitor.

8. Use of compounds of the formula I according to Claim 1 and/or physiologically acceptable salts thereof for the preparation of a medicament.

## Revendications

1. Composés répondant à la formule I 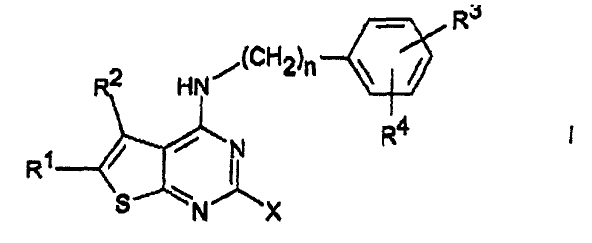 dans laquelle
R¹ et R² représentent chacun, indépendamment l'un de l'autre, A ou Hal,
R¹ et R² représentent ensemble alternativement alkylène ayant de 3 à 5 atomes de carbone,
R³ et R⁴ représentent chacun, indépendamment l'un de l'autre, H, OA ou Hal,
R³ et R⁴ représentent ensemble alternativement -O-CH₂-O- ou -O-CH₂-CH₂-O-,
A représente alkyle ayant de 1 à 6 atomes de carbone,
X représente 1-imidazolyle, 2-méthylimidazolyle, pyrazolyle, 1,2,4-triazol-1-yle ou 3-pyridyle,
Hal représente F, Cl, Br ou I,
et
n vaut 1,
à condition que si
R¹ et R² représentent chacun, indépendamment l'un de l'autre, A ou Hal,
alors R³ et R⁴ ne représentent pas H,
et les sels physiologiquement acceptables de ceux-ci.

2. Composés répondant à la formule I selon la revendication 1
(a) la 2-(1-imidazolyl)-5,6-diméthyl-4-(3,4-méthylènedioxybenzylamino)thiéno-[2,3-d]-pyrimidine;
(b) la 2-(1-imidazolyl)-4-(3,4-méthylènedioxybenzylamino)-5,6,7,8-tétrahydro-[1]-benzothiéno-[2,3-d]-pyrimidine;
(c) la 2-(1,2,4-triazol-1-yl)-4-(3,4-méthylènedioxybenzylamino)-5,6,7,8-tétrahydro-[1]-benzothiéno-[2,3-d]-pyrimidine;
(d) la 2-(pyrazol-1-yl)-4-(3,4-méthylènedioxybenzylamino)-5,6,7,8-tétrahydro-[1]-benzothiéno-[2,3-d]-pyrimidine;
(e) la 2-(pyridin-3-yl)-4-(3,4-méthylènedioxybenzylamino)-5,6,7,8-tétrahydro-[1]-benzothiéno-[2,3-d]-pyrimidine.

3. Procédé de préparation de composés répondant à la formule I selon la revendication 1 et de sels de ceux-ci, dans laquelle X est lié par l'intermédiaire de C,
**caractérisé en ce qu'**un composé répondant à la formule III 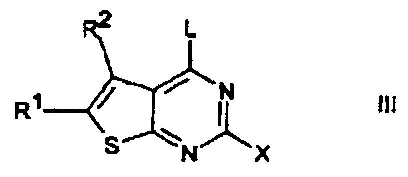 dans laquelle
R¹, R² et X ont les significations indiquées,
et L représente Cl, Br, OH, SCH₃ ou un groupement OH estérifié réactif,
est réagi avec un composé répondant à la formule IV 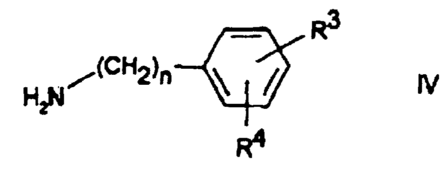 dans laquelle
R³, R⁴ et n ont les significations indiquées,
et/ou **en ce qu'**un composé basique répondant à la formule I est converti en l'un de ses sels par traitement par un acide.

4. Procédé de préparation de préparations pharmaceutiques, **caractérisé en ce qu'**un composé répondant à la formule I selon la revendication 1 et/ou un sel physiologiquement acceptable de celui-ci est mis sous une forme de dosage convenable conjointement avec au moins un excipient ou adjuvant solide, liquide ou semi-liquide.

5. Préparation pharmaceutique, **caractérisée par** une teneur en au moins un composé répondant à la formule I selon la revendication 1 et/ou un sel physiologiquement acceptable de celui-ci.

6. Composés répondant à la formule I selon la revendication 1 et les sels physiologiquement acceptables de ceux-ci pour lutter contre des maladies du système cardiovasculaire et pour la thérapie des troubles de la virilité.

7. Médicament répondant à la formule I selon la revendication 1 et les sels physiologiquement acceptables de celui-ci comme inhibiteur de la phosphodiestérase V.

8. Utilisation de composés répondant à la formule I selon la revendication 1 et/ou de sels physiologiquement acceptables de ceux-ci pour la préparation d'un médicament.
